**Europäisches Patentamt**

**European Patent Office**

**Office européen des brevets**

(11) Veröffentlichungsnummer: **0 425 892 A2**

# (12) EUROPÄISCHE PATENTANMELDUNG

(21) Anmeldenummer: 90119879.6

(22) Anmeldetag: 17.10.90

(51) Int. Cl.5: **A61K 9/14**

(30) Priorität: 28.10.89 DE 3936053

(43) Veröffentlichungstag der Anmeldung:
08.05.91 Patentblatt 91/19

(84) Benannte Vertragsstaaten:
AT BE CH DE DK ES FR GB GR IT LI NL SE

(71) Anmelder: **BASF Aktiengesellschaft**
**Carl-Bosch-Strasse 38**
**W-6700 Ludwigshafen(DE)**

(72) Erfinder: **Horn, Dieter, Dr.**
**Schroederstrasse 69**
**W-6900 Heidelberg(DE)**
Erfinder: **End, Lutz, Dr.**
**Tannhäuser Ring 103-105**
**W-6800 Mannheim 24(DE)**
Erfinder: **Spengler, Reinhard, Dr.**
**Comeniusstrasse 6**
**W-6700 Ludwigshafen(DE)**
Erfinder: **Krause, Hans-Jürgen, Dr.**
**Kopernikusstrasse 61**
**W-6520 Worms 26(DE)**
Erfinder: **Elzner, Jürgen, Dr.**
**Im Biengarten 37**
**W-6730 Neustadt 14(DE)**

(54) **Verfahren zur Verbesserung der Bioverfügbarkeit von pharmazeutischen Wirkstoffen mit Peptidbindungen.**

(57) Peptidbindungen enthaltende und daher empfindliche pharmazeutische Wirkstoffe werden gegen Abbau bei der Magen/Darm-Passage durch Mikronisierung geschützt. Dadurch wird die Resorption und Bioverfügbarkeit verbessert. Bei der Mikronisierung werden die Wirkstoffe feinst verteilt und jedes Teilchen mit einer Kolloid-Schutzhülle umgeben.

EP 0 425 892 A2

# VERFAHREN ZUR VERBESSERUNG DER BIOVERFÜGBARKEIT VON PHARMAZEUTISCHEN WIRKSTOFFEN MIT PEPTIDBINDUNGEN

Peptidbindungen enthaltende und daher empfindliche pharmazeutische Wirkstoffe werden erfindungsgemäß durch Mikronisierung gegen Abbau, vor allem bei der Magen/Darm-Passage, geschützt. Dadurch wird die Resorption und Bioverfügbarkeit verbessert. Bei der Mikronisierung werden die Wirkstoffe feinst verteilt und jedes Teilchen mit einer Kolloid-Schutzhülle umgeben.

Pharmazeutische Wirkstoffe mit Peptidbindungen sind bei der Magen/Darm-Passage in der Regel instabil. Deshalb müssen relativ große Mengen Wirkstoff eingenommen werden, um ausreichende Plasmaspiegel zu erreichen. Eine magensaftresistente Beschichtung stellt keine Lösung des Problems dar, weil die Peptidbindungen auch im Darm rasch abgebaut werden. Eine relativ komplexe Lösung ist in Science 233 , 1081(1986) von M. Saffran und G.S. Kumar beschrieben. Dabei wird jedoch die Resorption in unerwünschter Weise verzögert. Außerdem sind die dort eingesetzten Resorptionsbeschleuniger physiologisch nicht unbedenklich.

Der Erfindung lag die Aufgabe zugrunde, trockene pharmazeutische Präparate mit Peptidbindungen enthaltenden Wirkstoffen zu entwickeln, die bei der Magen/Darm-Passage stabiler als die üblichen sind und daher in höherem Maße resorbiert werden.

Die Lösung dieser Aufgabe besteht in einer Mikronisierung des Wirkstoffs, d.h. man löst ihn zusammen mit einem Tensid in einem flüchtigen, mit Wasser mischbaren organischen Lösungsmittel bei Temperaturen zwischen 5 und 200° C, gegebenenfalls unter erhöhtem Druck, innerhalb einer Zeit von weniger als 10 Sekunden, fällt aus der erhaltenen moleculardispersen Lösung sofort durch schnelles Mischen mit einer wäßrigen Lösung eines quellbaren Kolloids bei Temperaturen zwischen 0 und 50° C den Wirkstoff in kolloid-disperser Form aus und befreit die erhaltene Dispersion in an sich bekannter Weise von dem Lösungsmittel und dem Dispergiermedium.

Das Mikronisierverfahren ist für Carotinoide und Retinoide in der EP-A-65 193 zur Herstellung von Lebensmittel- und Futtermittel-Farbstoffen beschrieben. Es war nicht zu erwarten, daß dieses Verfahren zur Lösung der hier zugrundeliegenden Aufgabe herangezogen werden könnte. Überraschenderweise sind die erfindungsgemäß mikronisierten Wirkstoffe trotz ihrer extremen Feinverteilung gegen hydrolytischen oder enzymatischen Abbau der Peptidbindungen stabil. Die Wirkstoffe werden außerdem rasch und weitgehend resorbiert, so daß bereits relativ geringe Gaben verhältnismäßig hohe Plasmaspiegel bewirken.

Beispiele für Wirkstoffe mit Peptidbindungen sind Doreptide; Polypeptid-Antibiotika wie Tyrothricin, Polymyxin B; TNF; Immunmodulatoren wie Interferon-γ, Renin-Inhibitoren, ACE-Hemmer.

Beispiele für geeignete wassermischbare flüchtige organische Lösungsmittel sind Alkohole wie Ethanol, n-Propanol, iso-Propanol, Ether wie 1,2-Butandiol-1-methylether oder 1,2-Propandiol-1-n-Propylether und Ketone wie Aceton. Sie sollen zu mindestens 10 % wassermischbar sein, unter 200° C sieden und weniger als 10 Kohlenstoffatome enthalten. Beispiele für geeignete wasserlösliche oder quellbare Kolloide sind Gelatine, Stärke, Dextrin, Dextran, Pektin, Gummiarabikum, Kasein, Kaseinat, Vollmilch, Magermilch, Milchpulver, Polyvinylalkohol, Polyvinylpyrrolidon, Methylcellulose, Carboxymethylcellulose, Hydroxypropylcellulose, mikrokristalline Cellulose, Alginate. Bezüglich näherer Einzelheiten zu den Kolloiden wird auf R.A. Morton, Fast Soluble Vitamins, Intern. Encyclopedia of Food and Nutrition, Band 9, Pergamon Press 1970, Seiten 128-131, verwiesen. Zur Erhöhung der mechanischen Stabilität des Endproduktes kann man dem Kolloid einen Weichmacher zusetzen, beispielsweise Zucker wie Saccharose, Glucose, Lactose, Invertzucker, oder Zuckeralkohole, wie Sorbit oder Mannit, oder Maltodextrin oder Glycerin. Als Konservierungsstoffe können untergeordnete Mengen an z.B. Methylparaben, Propylparaben, Sorbinsäure und/oder Na-Benzoat zugefügt werden.

Als Tenside (Dispergiermittel) kommen beispielsweise in Betracht: Ester langkettiger Fettsäuren mit Zitronensäure, Milchsäure, Diacetylweinsäure oder Ascorbinsäure, insbesondere Ascorbylpalmitat, Mono- und Diglyceride von Fettsäuren und deren Oxethylierungsprodukte, Polyglycerinfettsäureester (z.B. das Monostearat des Triglycerins), Sorbitanfettsäureester, Propylenglykol-Fettsäureester, 2-(2′-stearoyllactyl)-milchsaure Salze und Lecithin. Je nach Löslichkeit wird das Tensid entweder im organischen Lösungsmittel (zusammen mit dem Wirkstoff) oder in der wäßrigen Phase gelöst.

Weitere galenische Hilfsmittel wie Bindemittel, Sprengmittel, Geschmacksstoffe, Vitamine, Farbstoffe, Netzmittel, den pH-Wert beeinflussende Zusätze (vgl. H. Sucker et al, Pharmazeutische Technologie, Thieme-Verlag, Stuttgart 1978) können ebenfalls über das Lösungsmittel oder die wäßrige Phase eingebracht werden. Es versteht sich von selbst, daß alle galenischen Hilfsmittel physiologisch unbedenklich sein müssen.

Das Verhältnis Kolloid und Weichmacher zu Wirkstoff- und Tensidlösung wird im allgemeinen so gewählt, daß ein Endprodukt erhalten wird, das zwischen 0,5 und 40, vorzugsweise etwa 20 Gew.-% Wirkstoff, 0,1 bis 30, vorzugsweise 5 bis 15 Gew.-% eines oder mehrerer Tenside, 10 bis 50 Gew.-% eines quellbaren Kolloids, 0 bis 70 Gew.-% eines Weichmachers, alle Prozentangaben bezogen auf die Trockenmasse des Pulvers, sowie gegebenenfalls untergeordnete Mengen eines Stabilisators enthält, wobei das Pulver eine mittlere Teilchengröße des Wirkstoffs von weniger als 0,8 μm und eine Halbwertsbreite der Korngrößenverteilung von weniger als 50 % und praktisch keine Anteile mit einer Korngröße über 1 μm aufweist.

Als Beispiele für geeignete Stabilisatoren seien genannt α-Tocopherol, t-Butyl-hydroxy-toluol, t-Butylhydroxyanisol und Ethoxyquine. Sie können (wie das Tensid) je nach Löslichkeit entweder der wäßrigen oder der Lösungsmittelphase zugesetzt werden.

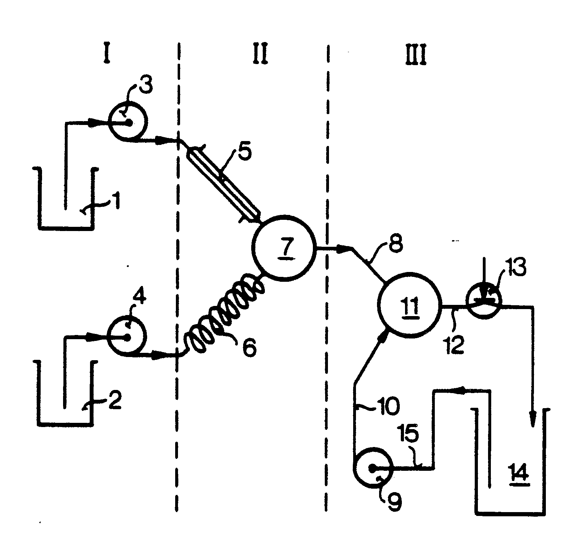

Im einzelnen führt man das erfindungsgemäße Verfahren beispielsweise mit einer Apparatur, wie sie in Abb. 1 schematisch dargestellt ist, wie folgt durch:

Die Apparatur gliedert sich in die Teile I, II und III. Teil II ist gegebenenfalls der Hochtemperaturbereich. In den Teilen I und III betragen die Temperaturen im allgemeinen weniger als 50° C.

Im Gefäß (1) wird eine Suspension des Wirkstoffes im Lösungsmittel und ggf. ein oder mehrere Tenside, gegebenenfalls gemeinsam mit einem geringen Zusatz von Stabilisatoren, vorgelegt. Eine besonders rasche Auflösung von temperaturempfindlichen oder bei Raumtemperatur schwerlöslichen Wirkstoffen kann durch vorhergehendes Mahlen (Teilchengröße <50 μm) erreicht werden. Gefäß (2) enthält das Lösungsmittel ohne Beimischung des Wirkstoffes. Über die Pumpen (3) bzw. (4) werden die Wirkstoffsuspension und das Lösungsmittel der Mischkammer zugeführt, wobei das Mischungsverhältnis durch Wahl der jeweiligen Förderleistung der Pumpen vorgegeben werden kann und so gewählt wird, daß je nach Löslichkeit des Wirkstoffes im Lösungsmittel und angestrebter Verweilzeit eine Wirkstoffkonzentration in der Mischkammer von 0,5 bis 10 Gew.-%, bezogen auf die Lösung, entsteht. Das Mischkammervolumen (7) wird bei thermolabilen Wirkstoffen vorzugsweise so bemessen, daß bei der eingestellten Förderleistung der Pumpen (3) und (4) die Verweilzeit in (7) vorzugsweise weniger als 1 Sekunde beträgt. Das Lösungsmittel wird vor Eintritt in die Mischkammer über den Wärmeaustauscher (6) auf die gewünschte Temperatur gebracht, während die Wirkstoffsuspension durch Zuführung über die (bei wärmeempfindlichen Wirkstoffen thermisch isolierte) Zuleitung (5) bei Temperaturen unter 50° C gehalten wird. Durch turbulente Mischung in (7) erfolgt im Temperaturbereich von 10 bis 240° C, vorzugsweise bei 100 bis 200° C (bei Wirkstoffen, die bei Raumtemperatur selbst im besten Lösungsmittel nur wenig löslich sind), die gemeinsame Lösung des Wirkstoffes und des Stabilisators, und die erhaltene Lösung tritt über den Überlauf (8) in die zweite Mischkammer (11) ein, in der durch Zumischen einer wäßrigen Schutzkolloid-Weichmacherlösung, die auch das Tensid enthält, falls dieses nicht im organischen Lösungsmittel gelöst wurde, über die Pumpe (9) und die Zuleitung (10) aus der molekulardispersen Wirkstofflösung und der wäßrigen Phase eine mikrodisperse Wirkstoffdispersion (Mikronisat) gebildet wird. Über Leitung (12) wird dann die Dispersion über das Überdruckventil (13) ausgetragen und im Vorratsgefäß (14) gesammelt. Zur Erzielung einer möglichst hohen Wirkstoffkonzentration kann die Dispersion über die Saugleitung (15) im Kreis geführt werden.

Bei Einstellung des Überdruckventils (13) auf Drücke oberhalb 1 bar können in dem neuen Verfahren sogar Lösungsmittel bei Temperaturen oberhalb ihres Siedepunktes (bei Normaldruck) verwendet werden.

Aus der Dispersion kann ein pulverförmiges Präparat in an sich bekannter Weise, z.B. gemäß den Angaben der DE-A 25 34 091, beispielsweise durch Sprühgranulation, Sprühtrocknen oder durch Sprühkühlen und Einhüllen der Teilchen, Abtrennen und Trocknen im Wirbelbett erfolgen.

Zum Sprühtrocknen wird die Dispersion entweder zuerst vom Lösungsmittel durch Destillation, vorzugsweise unter vermindertem Druck, oder durch Extraktion mit einem mit Waser nicht mischbaren Lösungsmittel befreit, oder die gesamte Mischung wird sprühgetrocknet und so Wasser und Lösungsmittel zusammen im Sprühturm entfernt.

An der Austragstelle des Sprühturms fällt das Wirkstoffpulver meist trocken und rieselfähig an. In manchen Fällen kann es zweckmäßig sein, die vollständige Trocknung zusätzlich in einem Wirbelbett vorzunehmen.

Anstelle der Herstellung der Pulverzubereitung durch Sprühtrocknung können beliebige andere Methoden angewandt werden, um die in der Wasser/Wirkstoff-Dispersion bereits feinverteilten Wirkstoffe in die Pulverform zu überführen. Ein bekanntes Verfahren, das im Falle gelierfähiger Schutzkolloide und Hilfsstoffe hier Anwendung finden kann, besteht z.B. darin, die Dispersion vom Lösungsmittel zu befreien und in Paraffinöl zu einer W/O-Emulsion zu emulgieren, die Emulsionströpfchen durch Kühlung zu gelieren, das Paraffin von den Teilchen abzutrennen, das erhaltene Material mit Benzin zu waschen und im Wirbelbett zu trocknen. Ebenso läßt sich durch Koazervation und anschließende Filtration eine Aufkonzentration der Wirkstoffdispersion erreichen.

Man erhält in jedem Fall ein Trockenpulver, das erneut in Wasser unter Erzielung einer gleichmäßigen Feinverteilung des Wirkstoffs im Korngrößenbereich unter 1 μm gelöst oder redispergiert werden kann.

Die erfindungsgemäß erhältlichen Pulver können galenisch in konventioneller Weise in folgende Formen gebracht werden: Tabletten, Filmtabletten, Dragees, Kapseln, Instant-Pulver, Trockensaft. Außerdem können sie als Zwischenprodukte bei der Herstellung von Lyophilisaten und Injektionslösungen dienen.

Bestimmung der Bioverfügbarkeit am Hund:

Die mikronisierte Substanz (Doreptide) wird Hunden oral (Dosis 50 mg/kg) mittels Schlundsonde appliziert, und den Tieren wird zu definierten Zeitpunkten Blut entnommen. Nach Gewinnung des Plasmas werden die Proben sofort tiefgefroren und zu einem späteren Zeitpunkt mittels HPLC analysiert (reverse phase, Fluoreszenzdetektion nach Derivatisierung). Die so erhaltenen Plasmaspiegel werden mit den Plasmaspiegeln, die bei gleicher Dosierung mit den gleichen Hunden (wash out phase 1 Woche) nach Gabe von in üblicher Weise hergestellten Doreptide-Tabletten erhalten werden, verglichen.

Beispiel 1

12 g L-Prolyl-2-phenyl-L-2-aminobutanoylglycinamid (Doreptide) wurden unter kräftigem Rühren in einer Mischung aus 2,4 g Ascorbylpalmitat und 40 g Isopropanol suspendiert und bei Einstellung des Druckbegrenzerventils (13) auf 25 bar in der Mischkammer (7) mit Isopropanol gemischt, das im Wärmetauscher (6) auf 200° C erhitzt worden war. Bei einer Dosierleistung von 0,26 l/h auf der Suspensionsseite und 0,38 l/h auf der Lösungsmittelseite betrug die Verweilzeit in der Mischkammer (7) 0,6 Sekunden. Die dabei entstandene molekulardisperse Lösung wurde sodann der Mischkammer (11) zugeführt, in der durch turbulentes Mischen mit einer mit 1N NaOH auf pH 11 eingestellten Lösung von 15 g Gelatine und 22,5 g Saccharose pro Liter eine Dispersion von Doreptide entstand. Im Auffanggefäß (14) betrug die Temperatur der Dispersion 30° C. Die Teilchengrößenanalyse durch Photonenkorrelationsspektroskopie (nach B. Chu, Laser Light Scattering Analysis, Acedemic Press, New York 1974) lieferte einen Wert für den mittleren Teilchendurchmesser von 370 nm bei einer Verteilungsbreite von +/- 60 %.

Durch Sprühtrocknung wurde ein gut handhabbares, wasserlösliches Trockenpulver erhalten, dessen Doreptidgehalt bei 16,5 % lag. Die Wiederauflösung des Trockenpulvers in kaltem Wasser ergab erneut eine Dispersion mit einer mittleren Teilchengröße von 300 nm +/- 55 %.

Beispiel 2

10 g Gramicidin S wurden in einer Mischung von 1,2 g Ascorbylpalmitat und 40 g Isopropanol suspendiert und in gleicher Weise wie in Beispiel 1 mikronisiert. Das Mikronisat entsprach in der Teilchengrößenverteilung der Wirkstoffdispersion dem Mikronisat aus Beispiel 1.

Beispiel 3

11 g N-[(1-ethyl-2-pyrrolidinyl)methyl]-2-methoxy-5-sulfamoyl-benzamid (Sulpirid) wurden in einer Mischung von 2,0 g Ascorbylpalmitat und 40 g Methanol suspendiert und in gleicher Weise wie in Beispiel 1 mikronisiert. Die mittlere Teilchengröße des Mikronisates lag bei 390 nm +/- 40 %.

Beispiele 4 bis 11

In gleicher Weise wie im Beispiel 1 beschrieben, lassen sich folgende, in Tabelle 1 aufgeführte, Wirkstoffe zu Mikronisaten verarbeiten, deren physikalisch-chemische Eigenschaften mit denen des Mikronisates aus Beispiel 1 übereinstimmen.

Tabelle 1

| Beispiel | Wirkstoff |
|---|---|
| 4 | Tyrothricin; Merck Index 9640 (10. Ausgabe) |
| 5 | Polymyxin B der Firma Fluka, D 7910 Neu-Ulm |
| 6 | 1-(3-Mercapto-2-methyl-1-oxopropyl)-L-prolin (Captopril) |
| 7 | 1-[N-[(S)-1-Carboxy-3-phenylpropyl]-L-alanyl]-L-prolin 1'-ethylestermaleat (Enalaprilmaleat) |
| 8 | 2(S)-[N-(Morpholinocarbonyl)-L-phenylalanyl-N$^\alpha$-methyl-L-histidylamino]-1-cyclohexyl-3(S)-hydroxy-6-methylheptan |
| 9 | N-Butyl-6-cyclohexyl-4-hydroxy-2-isopropyl-5-[N-[2-(3,3-dimethyl-2-oxobutyl)-3-phenylpropanoyl]-L-histidylamino]hexanoylamid |
| 10 | [N-(3-Amino-3-methyl-1-oxobutyl)-4-methoxy-L-phenylalanyl]-N-[(1S,2R,3S)-1-(cyclohexylmethyl)-2,3-dihydroxy-5-methylhexyl]-L-histidinamid |
| 11 | 2-Acetamido-3-O-[(R)-1-[[(S)-1-[[(R)-3-carbamoyl-1-carboxypropyl]carbamoyl]ethyl]carbamoyl]ethyl]-2-deoxy-D-glucopyranose butylester (Murabutide) |

EP 0 425 892 A2

## Ansprüche

1. Verfahren zur Verbesserung der Bioverfügbarkeit von pharmazeutischen Wirkstoffen mit Peptidbindungen, dadurch gekennzeichnet, daß man den jeweiligen Wirkstoff und ggf. ein Tensid in einem flüchtigen, mit Wasser mischbaren organischen Lösungsmittel bei Temperaturen zwischen 5 und 200°C, gegebenenfalls unter erhöhtem Druck, innerhalb einer Zeit von weniger als 10 Sekunden löst, aus der erhaltenen molekulardispersen Lösung sofort durch schnelles Mischen mit einer wäßrigen Lösung oder Dispersion eines löslichen oder quellbaren Kolloids (und eines Tensids, falls dieses nicht schon in der organischen Phase gelöst wurde) bei Temperaturen zwischen 0 und 50°C den Wirkstoff in kolloid-disperser Form ausfällt, und die erhaltene Dispersion in ein redispergierbares Pulver überführt, indem man sie in an sich bekannter Weise von dem Lösungsmittel und dem Dispergiermedium befreit.

2. Verfahren nach Anspruch 1, dadurch gekennzeichnet, daß die Komponenten in solchen Mengen eingesetzt werden, daß ein Pulver folgender Zusammensetzung entsteht:

0,5 bis 40 Gew.-% Wirkstoff

0,1 bis 30 Gew.-% Tensid

10 bis 50 Gew.-% eines quellbaren Kolloids

0 bis 70 Gew.-% Weichmacher

0 bis 70 Gew.-% eines oder mehrerer üblicher galenischer Hilfsmittel.